## Europäisches Patentamt

## European Patent Office

## Office européen des brevets

(1) Publication number: **0 092 060**
**B1**

(12) **EUROPEAN PATENT SPECIFICATION**

(45) Date of publication of patent specification: **05.11.86**

(51) Int. Cl.⁴: **A 61 K 9/24**

(21) Application number: **83102781.8**

(22) Date of filing: **21.03.83**

(54) **Pharmaceutical products in sustained-release form, and their preparation process.**

(30) Priority: **15.04.82 IT 2075882**

(43) Date of publication of application:
**26.10.83 Bulletin 83/43**

(45) Publication of the grant of the patent:
**05.11.86 Bulletin 86/45**

(84) Designated Contracting States:
**AT BE CH DE FR GB LI LU NL SE**

(56) References cited:
**FR-A-2 148 045**
**FR-A-2 223 047**
**FR-A-2 419 722**
**US-A-4 252 786**

**DICTIONNAIRE VIDAL, 56th edition, 1980,
Vulg. Pharmaceutique, Paris, FR. "Tiapridal",
page 1264**

**UNLISTED DRUGS, vol. 32, no. 10, October
1980, page 156: J, Chatman, USA "Italprid"
UNLISTED DRUGS, vol. 33, no. 7, July 1981,
page 117: d, Chatham, USA "Tildiem"**

(73) Proprietor: **LABORATORI PROPHIN Industria
Chimica Farmaceutica S.p.A.
Via Binda, 21
Milan (IT)**

(72) Inventor: **Colombo, Paolo
Via Magenta, 12
Pavia (IT)**
Inventor: **Conte, Ubaldo
Via Strada Persa 7/B
Pavia (IT)**

(74) Representative: **Gervasi, Gemma, Dr. et al
Studio Brevetti e Marchi NOTARBARTOLO &
GERVASI 33, Viale Bianca Maria
I-20122 Milano (IT)**

Courier Press, Leamington Spa, England.

## Description

This invention relates to pharmaceutical sustained release products and the process for their preparation.

The problem of preparing sustained-release forms for oral administration is widely felt in the pharmaceutical industry.

A sustained-release form is a pharmaceutical form which makes the active principle available to the organism at a rate which is as constant as possible over a sufficiently long time period.

In practice, this implies finding the means for covering the organism with the drug over the 24 hours without using too frequent administration, which could be poorly tolerated by the patient, including phsychologically.

It also implies preventing large fluctuations in the hematic levels, with corresponding very high values which can induce undesirable side-effects. This type of problem is particularly felt in the case of drugs with a short half-life.

Various sustained-release forms are either proposed in the literature or have been prepared in practice, but always for specific drugs, with very limited objectives, and results which are only partly satisfactory.

U.S. Patent 4.252.786 describes medicinal tablets comprising a polymeric matrix and a polymeric film coating the matrix, said polymeric film comprising a polymeric component water soluble in an acidic medium (under pH 5,5).

French Patents 2.419.722 and 2.148.045 are relevant to the preparation of microcapsules of the type gastro-resistant and entero-soluble: in this case the film coating is soluble at a pH higher than 5 and insoluble and impermeable at a pH lower than 5.

A completely new form for oral administration has now been found, and forms the subject matter of the present invention, which enables a wide class of drugs to be administered in sustained-release form, independently of their chemical structure or type of activity.

More specifically, the administration form according to the present invention enables a single dose of drug to be introduced into the organism and to be released at a constant determined rate over a programmed time.

The new pharmaceutical product in sustained-release form is characterized by the following essential components:

a) a powder drug composition which produces an acid pH not exceeding 5 when in aqueous solution;

b) a nucleus constituted by a continuous rigid polymer matrix insoluble in water at pH<5 and biodegradable at pH exceeding 5, which polymer matrix contains the drug composition; and is selected from cellulose acetophtalate, polyvinyl acetophtalate, methylcellulose phtalate and hydroxypropylmethylcellulose phtalate;

c) a first film applied over the nucleus which comprises a film forming acrylic polymer permeable to the drug in aqueous solution, insoluble in water at any pH value;

d) a second film applied over the first film which comprises a film forming acrylic polymer having permeability to the drug not less than that of first film, insoluble but swellable in water.

In particular the embodiments of the present invention are based on the following functional requirements:

a) the nucleus which contains the drug is constituted by a polymer arranged to incorporate the drug and any excipients or diluents, and is in the form of a rigid continuous matrix insoluble at acid pH but soluble practically totally at pH values exceeding 5;

b) the first film covering the nucleus and constituted by a film forming acrylic polymer which is insoluble and not swellable in water, is chemically unalterable in an aqueous environment at physiological pH, and is absolutely free of toxicity.

The purpose of this first film is to regulate the release of the drug by means of a membrane effect. It must therefore be permeable to the drug and its thickness must vary as a function of the rate of diffusion of the drug. More specifically, this first film has a thickness, expressed in $g/cm^2$, which is directly proportional to the rate of diffusion of the drug expressed in $g/h$;

c) the second film, applied over the first film, and constituted by a film forming acrylic polymer which is insoluble in water at any pH, but is swellable, is free from toxicity and is totally permeable to the drug. The purpose of this second film, which must in no way obstruct or regulate the release of the drug, is purely to protect the first film so as to prevent any slight surface damage altering the completeness and consequently modifying the regularity of the membrane effect.

In this respect, it should be noted that the new sustained-release form according to the present invention utilises the membrane diffusion principle goverened by the first Fick law:

$$-\frac{dQ}{dt} = AS\ (C_1 - C_2)$$

where

$$-\frac{dQ}{dt}$$

is the diffusion rate, A is the diffusion constant, S is the membrane surface area, $C_1$ is the drug concentration on the inside of the membrane, and $C_2$ is the drug concentration on the outside of the membrane.

To enable the objects of the invention to be attained, the rate of diffusion of the drug must be as constant as possible, the drug quantity made available to the organism in unit time being consequently constant. Considering the aforesaid equation, it is apparent that as $C_2$ is negligible, because of the fact that the drug is continuously removed from the outside of the membrane, the proposed system will attain its object if S and $C_1$ are constant with time.

With regard to $C_1$, it has been found that by using a suitable film forming polymer for the first film applied on to a matrix, $C_1$ remains constant and equal to the concentration of the saturated aqueous solution of the drug, while this latter is present in the solid state within the matrix.

With regard to S, it has been found that when the drug is inserted into the rigid polymer matrix of point (a), this matrix creates a dimensionally stable support for the diffusive film of point (b), and thus maintains the surface area of the membrane constant for the entire required period.

In order to determine this period and make it coincide with the useful time period during which the drug can be absorbed by the organism, it has been found necessary to use polymers for preparing the matrix nucleus which are insoluble at acid pH, and in particular insoluble at pH values less than 5, but are totally soluble at pH values exceeding 5. In this manner, the nucleus containing the drug remains unaltered while in the presence of gastric juices of pH between 1.2 and 3, whereas it dissolves completely at the intestinal pH of between 5.5 and 7.5. The new system is constructed in such a manner that the dissolving of the rigid polymer matrix coincides with the exhaustion of the drug dose. The presence of a drug of acid hydrolysis pH ensures that the pH of the internal environment of the matrix is kept below the solubility limit of the polymer.

This facility for the nuclei to be totally biodegradable at intestinal pH is highly advantageous, because it prevents any residue accumulation at the intestinal level.

An important aspect of the present invention is that the essential characteristic of the polymers used in the nuclei in the new sustained-release form, i.e. their insolubility within the pH range of 1 to 5 or rather their diodegradability at pH values exceeding 5, also creates precise limits on the drugs which can be administered by the new system. In this respect, as already stated, the drugs administered with the new formulation must give a pH of between 1 and 5 in saturated aqueous solution in order to prevent the nuclei dissolving.

Drugs which have a higher pH can also be administered, provided they can be used in mixture with pharmaceutically acceptable acid substances such as tartaric acid, citric acid and the like, which maintain the pH of the system at the required value.

The preparation process for the new pharmaceutical forms comprises the following essential stages:

1. Preparation of the nuclei using polymers soluble in non-toxic organic solvents, such as alcohol, ketones, ethers and the like, and selected from cellulose acetophthalate, polyvinyl acetophthalate, methylcellulose phthalate and hydroxypropylmethylcellulose phthalate.

The solvent used must preferably not dissolve the drug.

The active principle in powder form, possibly mixed with diluents and excipients, is wetted with a polymer solution to such an extent that the weight of polymer is 10—20% of the weight of active principle or of powder material generally.

The uniformly mixed wetted mass obtained in this manner is granulated and dried.

The granulate, possibly mixed with other excipients, is compressed into tablet form.

It is critical for the purpose of the invention that the ratio of the polymer to the other components and the method of preparation used are such as to enable a "continuous" matrix to be formed.

2. Application of the first film to the nuclei for controlling the drug diffusion in an aqueous environment.

For this stage, film forming acrylic polymers are used which are insoluble in water at any pH value, soluble in non-toxic organic solvents which do not dissolve the active principle.

Under acrylic polymers are intended acrylic polymers and acrylic copolymers.

This film is preferably applied in a pan by spraying the polymer solution on to the preformed nuclei. The film is applied in successive cold-dried layers until the required thickness expressed in $g/cm^2$ is obtained.

3. Application of the second protective film for protecting the first film. Film forming acrylic polymers soluble in no-toxic organic solvents and insoluble but swellable in water, are used for this stage. From the chemical aspect, the classes of polymers which is used for the second film are the same as used for forming the first film, i.e. acrylic polymers, but chosen so as to in no way obstruct the drug diffusion.

This film is also applied by spraying the polymer solution on to the properly dried nuclei coated with the first film.

In this case, the film thickness is not critical, and is in fact limited to the minimum necessary for obtaining the protective effect. The thus finished pharmaceutical forms are cold-dried and possibly covered with a film of talc or other substance of lubricant action. It has been found that the new system has an action time lag of one to two hours, i.e. a certain time passes before the films become permeated by the water and the uniform release of the drug commences.

If it is not convenient to leave the organism "uncovered" for such a long period, it is possible to incorporate into the outer second film a quantity of drug which is immediately released, so acting within the time period necessary for the sustained-release form to attain its release equilibrium.

If a time lag of one hour is determined, then a drug quantity is incorporated into the second film equivalent to the hourly dose released by the sustained-release form.

According to this alternative, the purpose of the second film is then not only to protect the first film, but to act as a carrier for the immediate release of the drug.

The drug is incorporated into the polymer of the second film before its application by various methods.

It is possible to use a solvent for the polymer which is also a solvent for the active principle, and to apply the film as a solution. It is also possible to prepare the drug in the form of an aqueous solution and use a solvent for the polymer which is able to totally mix with the drug solution.

In this case the second film is also applied in the form of a solution.

Excellent results have also been obtained by uniformly suspending the drug in powder form in the polymer solution, and applying the suspension in the described manner.

As already stated, the drugs which can be administered in the new sustained-release form are all those drugs which in aqueous solution give a pH of between 1 and 5, such as the hydrochlorides of weak bases or drugs which themselves contain acid groups. If basic drugs are to be administered, they can be put into the sustained-release form in mixture with a pharmaceutically acceptable acid substance.

A non-limiting example of the invention is given hereinafter using Tiapride, i.e. N - diethylaminoethyl - 2 - methoxy - 5 - methylsulphonyl - benzamide hydrochloride, which is normally administered in a daily dosage of $4\times100$ mg.

Example 1

The optimum hourly dose of Tiapride was calculated for a subject weighing 70 kg on the basis of pharmacokinetic parameters obtained in man as follows:

— half life 4 hours
— elimination constant (Kel):

$$\frac{0.693}{4}=0.173 \text{ h}^{-1}$$

—- hematic peak after administering 100 mg (Cp): 0.8 mg/l
— therapeutic range: 0.6—0.9 mg/l of plasma
— distribution volume (Vd):100 l.

Constant-rate release for a subject weighing 70 kg:

$$Cp\times\frac{Vd \cdot Kel}{W}=0.8\times\frac{100\times0.173}{70}=0.1977 \text{ mg/h/kg}$$

giving a total of $0.1977\times70=13.84$ mg/h.

The quantity of Tiapride necessary to keep the hematic level constant over 12 hours is $13.84\times12=166$ mg.

a) The following quantities of substances were used for preparing 1000 nuclei:

| | |
|---|---|
| — Tiapride hydrochloride | 166 g |
| — Cellulose acetophthalate biodegradable at pH 5 | 31 g |
| — talc | 100 g |
| — magnesium stearate | 3 g |

The Tiapride hydrochloride and talc were placed in a powder mixer and mixed for a sufficient time to give a completely homogeneous mixture.

A solution of the polymer was prepared in 60 ml of a 6:2 acetone:alcohol mixture, and this was used to wet the powder mixture.

The wetted mass was granulated through a mesh with 800 μm apertures, dried and repassed through a mesh with 500 μm apertures.

The granulate obtained was mixed with the magnesium stearate and compressed by using concave punches of 9 mm diameter at a pressure of 2000 kg/cm².

b) The following substances were used for the application of the first film for controlling the drug diffusion:

| | |
|---|---|
| — low permeability acrylic polymer | 5.04 g |
| — high permeability acrylic polymer | 1.26 g |
| — dimethylpolysiloxane | 1 g |
| — acetone | 25 ml |
| — isopropanol | 25 ml |

The film was applied to the nuclei in a copper pan, by spraying the film forming polymer solution in short bursts followed by pauses for drying with cold air. It was found that the weight increase had to be 7.3 g in order to obtain the necessary film thickness.

The weight increase was checked after drying the coated nucle in an oven until their weight was constant.

c) The following substances were used for the application of the second protective film:

| | |
|---|---|
| — high permeability acrylic polymer | 6.25 g |
| — dimethylpolysiloxane | 1 g |
| — acetone | 25 ml |
| — isopropanol | 25 ml |

The polymer solution was again applied in a pan to the dry coated nuclei originating from the preceding stage, using the method heretofore described.

A film thickness equivalent to a weight increase of about 5 g was applied.

After the second film had been applied, the nuclei were rolled in a drum lined with a minimum quantity of talc.

The Tiapride release was monitored for the uncoated nuclei, the nuclei with the first film applied, and the finished nuclei also comprising the second film.

A USP XX paddle apparatus was used, the dissolving medium being distilled water at 37°C.

The obtained results given hereinafter are the average of 6 determinations.

A— Tiapride release from the nuclei, expressed as a percentage of the total initial quantity:

| Time (min) | % Drug released |
| --- | --- |
| 15 | 37.8 |
| 30 | 51.6 |
| 60 | 71.1 |
| 90 | 80.3 |
| 120 | 86.0 |

B— Tiapride release from the nuclei covered with the first film:

| Time (hours) | % Drug released |
| --- | --- |
| 1 | 2 |
| 2 | 4 |
| 3 | 14 |
| 3.5 | 19.3 |
| 4.5 | 34 |
| 5 | 47 |
| 6 | 57 |
| 7 | 69 |
| 8 | 83 |

C— Tiapride release from the nuclei covered with the first and second films:

| Time (hours) | % Drug released |
| --- | --- |
| 1 | 0 |
| 2 | 1 |
| 3 | 16 |
| 4.5 | 34 |
| 5.5 | 44 |
| 6.5 | 57 |
| 7.5 | 71 |
| 8.5 | 83 |

As can be clearly seen from the reported data, when simply pasted into the polymer support matrix the drug is totally released within two hours, whereas if covered with the fiffusion regulating film it is released in practice within a time period of 9 to 10 hours, during which the release kinetics remain of zero order, with a release rate within the acceptable limits of 10—15 mg/h for a calculated optimum dose of 13.84 mg/h.

It is also apparent that the second film has practically no influence on the drug diffusion rate, but in fact provides improved release regularity practically until the drug is exhausted.

By using the new sustained-release form, the Tiapride dosage can be changed from 4×100 mg to 2×166 mg over the 24 hours.

Example 2

The optimum hourly dose of Diltiazem was calculated for a subject weighing 70 kg and based on pharmacokinetic parameters obtained from humans as follows:

— half life 4 hours
— elimination constant (Kel):

$$\frac{0.693}{4}=0.123 \ h^{-1}$$

— hematic peak after administration of 60 mm (Cp): 0.072 mg/ml
— therapeutic range: 0.4—0.9 mg/l of plasma
— distribution volume (Vd): 1065

Constant-rate release for 70 kg subject:

$$Cp \times \frac{Vd \cdot Kel}{W}=$$

0.1347 mg/h/kg for a total of 0.1977×70=9.43 mg/h.

The quantity of Diltiazem for maintaining a constant hematic level over a 12-hour period is 9.43×12=115 mg (rounded off to the high side).

a) The preparation of 1000 nuclei requires the following product amounts:

| | |
| --- | --- |
| — Diltiazem hydrochloride | 115 g |
| — Biodegradable cellulose acetophthalate at pH 5 | 31 g |
| — Talc | 150 g |
| — Magnesium stearate | 3 g |

The Diltiazem hydrochloride and talc are put inside a powder mixer and mixed long enough to produce a completely homogeneous mixture.

A solution of polymer was prepared in 60 ml of 6:2 acetonealcohol mixture and was used to wet the powder mixture.

The wetted mass was granulated through a screen having an 800 μm mesh and then dried and repassed through a screen having a 500 μm mesh.

The obtained granulate was dried and mixed with the magnesium stearate, followed by compression under 9 mm dia. concave punches under a pressure of 2000 kg/cm².

b) The following products were used to apply the first film layer for controlling drug diffusion:

— low-permeability acrylic
    polymer                           5.04 g
— high-permeability acrylic
    polymer                           1.26 g
— dimethylpolysiloxane               1.00 g
— acetone                            25 ml
— isopropanol                        25 ml

The film was applied to the nuclei, held in a copper pan, by alternately spraying with film forming polymer solution in short bursts and drying with cold air. It was found that in order to obtain the required film thickness, the increase in weight of the entire lot had to be 7.3 g, which amounted to an increase of 7.3 mg for each nucleus.

The increase in weight was checked after drying the coated nuclei in an oven until the weight became constant.

c) The following products were used for applying the second protective film coating:

— high-permeability acrylic
    polymer                           6.25 g
— dimethylpolysiloxane               1 g
— acetone                            25 ml
— isopropanol                        25 ml

The film was applied to the nuclei, held in a copper pan, in the same manner as described above, using the polymer solution and the nuclei processed in the preceding phase.

A film coating was applied which amounted to an increase of weight of about 5 g.

After having applied the second coating, the nuclei were rolled in a drum having a minimum quantity of talc liner.

The Diltiazem release rate was measured on noncoated nuclei, nuclei coated with the first film and the finalized form with the second film coating.

A USP XX paddle apparatus was used and the dissolving medium was distilled water at 37°C.

The results shown below are the average values of 6 determinations:

A— Diltiazem release from the nuclei, expressed as per cent of the total initial quantity:

| Time (min) | % Release |
|-----------|-----------|
| 15 | 37.8 |
| 30 | 51.6 |
| 60 | 71.1 |
| 90 | 80.3 |
| 120 | 86.0 |

B— Diltiazem release from the nuclei with one layer of drug-release control film:

| Time (h) | % Release |
|----------|-----------|
| 1 | 3 |
| 2 | 10.3 |
| 3 | 16 |
| 3.5 | 19 |
| 4.5 | 35 |
| 5 | 48 |
| 6 | 59 |
| 7 | 70 |
| 8 | 86 |

C— Diltiazem release from the nuclei coated with the first and second films:

| Time (h) | % Release |
|----------|-----------|
| 1 | 2 |
| 2 | 8 |
| 3 | 16 |
| 4.5 | 35 |
| 5.5 | 46 |
| 6.5 | 58 |
| 7.5 | 72 |
| 8.5 | 84 |

As can clearly be seen from the above data, when the drug is simply mixed in with the polymer support matrix, it is totally released within two hours; whereas when it is covered with the diffusion regulating film, it is practically completely released within 9 to 10 hours, during which the release kinetics remain on the order of zero with the release rate remaining between the acceptable limits of between 10—15 mg/h for a calculated optimum dose of 9.43 mg/h.

It is also apparent that the second film coating has practically no effect on the diffusion rate, while it does provide for more release regularity almost until the drug is completely exhausted.

By using the new retarding formula, the Diltiazem dosage can be changed from 3×60 mg to 2×115 mg per 24 hour period.

## Claims

1. A pharmaceutical product in sustained-release form for oral administration comprising:

a) a powder drug composition which produces an acid pH not exceeding 5 when in aqueous solution;

b) a nucleus constituted by a continuous rigid polymer matrix insoluble in water at pH<5 and biodegradable at pH exceeding 5, which polymer matrix contains the drug composition; and is selected from cellulose acetophtalate, polyvinyl acetophtalate, methylcellulose phtalate and hydroxypropylmethylcellulose phtalate;

c) a first film applied over the nucleus which comprises a film forming acrylic polymer permeable to the drug in aqueous solution, insoluble in water at any pH value;

d) a second film applied over the first film which comprises a film forming acrylic polymer having permeability to the drug not less than that of first film, insoluble but swellable in water.

2. A pharmaceutical product according to claim 1, wherein the nucleus of item b) comprises 10—20% by weight of biodegradable polymer.

3. A pharmaceutical product according to claim 1, wherein the nucleus comprises 40—60% by weight of the drug.

4. A process for preparing a pharmaceutical product in sustained-release form according to claim 1, characterised in that (1) the active principle in powder form, possibly mixed with excipients and diluents, is wetted with a solution of the polymer which is biodegradable at pH values exceeding 5, and selected from cellulose acetophtalate, polyvinyl acetophtalate, methylcellulose phtalate and hydroxypropylmethylcellulose phtalate, then granulated and dried under cold conditions, (2) a solution of the film forming acrylic polymer insoluble in water is applied over the nuclei thus formed and the solvent is evaporated under cold conditions to form the first film, (3) a solution of the film forming acrylic polymer having permeability to the drug not less than that of the first film and which is insoluble but swellable in water is applied over the first formed film and the solvent is evaporated under cold conditions to form a second protective film.

5. A process as claimed in claim 4, characterised in that the solution of the biodegradable polymer is prepared using a non-toxic organic solvent which is not a solvent for the active principle.

6. A process as claimed in claim 4, characterised in that the first and second films are applied in the form of successive concentric layers applied on to the preformed nuclei until the required thickness is attained, by spraying a solution of polymer in a non-toxic organic solvent.

7. A process as claimed in claim 4, characterised in that before the second film polymer solution is applied, either a solution of active principle is dissolved therein or the active principle as such is suspended therein.

## Patentansprüche

1. Pharmazeutisches Produkt in einer Form mit verzögerter Freigabe für orale Verabreichung, umfassend:

a) eine pulverförmige Arzneimittelzusammensetzung, die einen sauren, 5 nicht übersteigenden pH ergibt, wenn sie in wässeriger Lösung ist,

b) einen Kern, der von einer kontinuierlichen starren Polymermatrix gebildet wird, die in Wasser bei pH<5 unlöslich ist und bei pH über 5 bioabbaubar ist, welche Polymermatrix die Arzneimittelzusammensetzung enthält und. ausgewählt ist aus Zelluloseacetophthalat, Polyvinylacetophthalat, Methylzellulosephthalat und Hydroxypropylmethylzellulosephthalat;

c) einen ersten Film, der über dem Kern aufgebracht ist, der ein filmbildendes Acrylpolymer umfaßt, das für das Arzneimittel in wässeriger Lösung durchlässig und in Wasser bei jedem pH-Wert unlöslich ist;

d) einen zweiten Film, der über dem ersten Film aufgebracht ist, der ein filmbildendes Acrylpolymer mit einer Durchlässigkeit für das Arzneimittel umfaßt, die nicht geringer ist als jene des ersten Films, und das in Wasser unlöslich, aber quellfähig ist.

2. Pharmazeutisches Produkt gemäß Anspruch 1, worin der Kern gemäß Punkt b) 10 bis 20 Gew.% des bioabbaubaren Polymers umfaßt.

3. Pharmazeutisches Produkt gemäß Anspruch 1, worin der Kern 40 bis 60 Gew.% des Arzneimittels umfaßt.

4. Verfahren zum Herstellen eines pharmazeutischen Produktes in einer Form mit verzögerter Freigabe gemäß Anspruch 1, dadurch gekennzeichnet, daß (1) der Wirkstoff in Pulverform, gegebenenfalls in Mischung mit Exzipienten und Verdünnungsmitteln, mit einer Lösung des Polymers angefeuchtet wird, das bei pH-Werten über 5 bioabbaubar ist und ausgewählt wird aus Zelluloseacetophthalat, Polyvinylacetophthalat, Methylzellulosephthalat und Hydroxypropylmethylzellulosephthalat, dann granuliert und unter kalten Bedingungen getrocknet wird, (2) eine Lösung des filmbildenden Acrylpolymers, das in Wasser unlöslich ist, auf die so gebildeten Kerne aufgebracht und das Lösungsmittel unter kalten Bedingungen unter Bildung des ersten Films abgedampft wird, und (3) eine Lösung des filmbildenden Acrylpolymers mit einer Durchlässigkeit für das Arzneimittel, die nicht geringer ist als jene des ersten Films, und das in Wasser unlöslich, aber quellfähig ist, auf den ersten gebildeten Film aufgebracht und das Lösungsmittel unter kalten Bedingungen unter Bildung eines zweiten Schutzfilms abgedampft wird.

5. Verfahren, wie in Anspruch 4 beansprucht, dadurch gekennzeichnet, daß die Lösung des bioabbaubaren Polymers unter Verwendung eines nicht-toxischen organischen Lösungsmittels hergestellt wird, das kein Lösungsmittel für den Wirkstoff darstellt.

6. Verfahren, wie in Anspruch 4 beansprucht, dadurch gekennzeichnet, daß der erste und der zweite Film in Form von aufeinanderfolgenden konzentrischen Schichten aufgebracht werden die auf die vorgeformten Kerne durch Aufsprühen einer Lösung des Polymers in einem nicht-toxischen organischen Lösungsmittel aufgebracht werden, bis die erforderliche Dicke erzielt ist.

7. Verfahren, wie in Anspruch 4 beansprucht, dadurch gekennzeichnet, daß vor dem Aufbringen der zweiten Filmpolymerlösung entweder eine Lösung des Wirkstoffes darin gelöst oder der Wirkstoff als solcher darin suspendiert wird.

**Revendications**

1. Produit pharmaceutique sous une forme à libération prolongée pour administration orale, comprenant:

a) une composition médicamenteuse en poudre qui produit un pH acide ne dépassant pas 5 lorsqu'elle est en solution aqueuse;

b) un noyau constitué par une matrice rigide continue de polymère insoluble dans l'eau à un pH inférieur à 5 et biodégradable à un pH dépassant 5, matrice de polymère qui contient la composition médicamenteuse et qui est choisie parmi l'acétophtalate de cellulose, l'acétophtalate de polyvinyle, le phtalate de méthylcellulose et le phtalate d'hydroxypropylméthylcellulose;

c) une première pellicule appliquée sur le noyau, comprenant un polymère acrylique filmogène qui est perméable à la drogue en solution aqueuse et insoluble dans l'eau, quelle que soit la valeur de pH;

d) une seconde pellicule appliquée sur la première pellicule, comprenant un polymère acrylique filmogène dont la perméabilité à la drogue n'est pas inférieure à celle de la première pellicule et qui est insoluble, mais susceptible de gonflement dans l'eau.

2. Produit pharmaceutique selon la revendication 1, dans lequel le noyau défini en b) comprend 10 à 20% en poids de polymère biodégradable.

3. Produit pharmaceutique selon la revendication 1, dans lequel le noyau comprend 40 à 60% en poids de la drogue.

4. Procédé pour la préparation d'un produit pharmaceutique sous une forme à libération prolongée suivant la revendication 1, caractérisé en ce que: (1) le principe actif sous forme de poudre, éventuellement mélangé avec des excipients et des diluants, est mouillé avec une solution du polymère qui est biodégradable à des valeurs du pH dépassant 5 et est choisi parmi l'acétophtalate de cellulose, l'acétophtalate de polyvinyle, le phtalate de méthylcellulose et le phtalate d'hydroxypropylméthylcellulose, puis il est granulé et séché, (2) une solution de polymère acrylique filmogène insoluble dans l'eau est appliquée sur le noyau ainsi formé et le solvant est évaporé à froid pour former la première pellicule, (3) une solution de polymère acrylique filmogène dont la perméabilité à la drogue n'est pas inférieure à celle de la première pellicule et qui est insoluble, mais susceptible de gonflement dans l'eau, est appliquée sur la première pellicule formée et le solvant est évaporé à froid, pour former une seconde pellicule protectrice.

5. Procédé selon la revendication 4, caractérisé en ce que la solution du polymère biodégradable est préparée en utilisant un solvant organique non toxique qui n'est pas un solvant pour le principe actif.

6. Procédé selon la revendication 4, caractérisé en ce que la première et la seconde pellicules sont appliquées sous la forme de couches concentriques successives appliquées sur les noyaux pré-formés jusqu'à ce que l'épaisseur requise soit atteinte, par pulvérisation d'une solution de polymère dans un solvant organique non toxique.

7. Procédé selon la revendication 4, caractérisé en ce qu'avant l'application de la solution de polymère de la seconde pellicule, une solution de principe actif y est dissoute ou le principe actif y est mis tel quel en suspension.